# DEMANDE DE BREVET EUROPEEN

(11) **EP 1 154 361 A1**
(43) Date de publication de la demande: **14.11.2001**
(21) Numéro de dépôt: 00402989.8
(22) Date de dépôt: 26.10.2000
(51) Int. Cl.: G06F 17/60, H04B 1/38, H04M 1/02

(54) **Procédé et dispositif d'édition d'une feuille de soin électronique**

(30) Priorité: 27.10.1999 FR 9913441
(71) Demandeur: SAGEM S.A., 75015 Paris (FR)
(72) Inventeur: Sarradin, Jean-Louis, 95190 Fontenay en Parisis (FR)
(74) Mandataire: Schmit, Christian Norbert Marie

(57) **Abrégé**

Pour remédier au problème de l'édition des feuilles de soin électroniques par des praticiens au cours de leurs déplacements chez leurs patients, on prévoit de munir le téléphone mobile (17) de ces praticiens d'un lecteur double (26) de cartes à puce. Ce lecteur double est capable de recevoir une carte à puce (28) d'un patient en même temps que la carte à puce (27) du praticien. Le lecteur double ou le téléphone mobile sont alors capable (38) d'une part d'éditer la feuille de soin électronique correspondant à la consultation en cours et d'autre part de la faire parvenir, par voie hertzienne (11) notamment, à un centre serveur (5) de données de sécurité sociale.

## Description

La présente invention a pour objet un dispositif d'édition d'une feuille de soin électronique. Elle est plus particulièrement destinée à être utilisée dans le domaine de l'administration de soins par des professionnels de santé. Les professionnels de santé concernés peuvent être des médecins, des pharmaciens, des masseurs kinésithérapeutes, des infirmières et d'une manière générale toute personne habilitée à intervenir dans ce domaine, et dont l'action conduit à un remboursement ou à un paiement indirect du prix de la prestation. Une feuille de soin électronique comporte comme indications essentielles les références d'un patient, éventuellement son nom mais surtout son numéro de sécurité sociale. Elle comporte les références du professionnel de santé, du praticien, qui a effectué l'acte, et le montant de la prestation. D'autres informations telles que l'heure, la date, ou la liste des médicaments d'une ordonnance peuvent également être incorporées à la feuille soin électronique.

Dans l'état de la technique, pour éditer de telles feuilles de soin électroniques, typiquement des fichiers informatiques, les praticiens disposent dans leur cabinet d'un lecteur double de cartes à puce. Ce lecteur double est appelé à recevoir une carte à puce du praticien, par exemple celle du médecin, et une carte à puce d'un patient reçu en consultation. Ce double lecteur est par ailleurs relié, notamment par l'intermédiaire d'un ordinateur personnel, à des moyens de transmission, en pratique un modem pour transmettre en temps réel, ou en temps différé, un fichier correspondant à la feuille de soin électronique. Ce fichier comporte les références du praticien prélevées dans la carte à puce praticien par l'intermédiaire du premier lecteur, les références du patient prélevées dans la carte à puce du patient prélevées par le deuxième lecteur, et le montant qui peut être toujours le même, ou modifié à l'aide d'un clavier prévu à cet effet dans ce double lecteur ou l'ordinateur personnel. En variante, le double lecteur peut être relié à une imprimante et à un système informatique spécialisé pour éditer les feuilles de maladie papier et/ou des ordonnances. Ces doubles lecteurs sont sécurisés dans leur fonctionnement par la présence de la carte à puce du praticien. Cette carte à puce de praticien lui est délivrée par un organisme de sécurité sociale. L'ensemble d'un tel système informatique est assez volumineux et son encombrement est sensiblement égal à celui d'un micro-ordinateur de type personnel, augmenté du volume de deux imprimantes du type à jet d'encre représentant le double lecteur et l'imprimante elle-même. Un tel équipement n'est bien entendu pas transportable.

Or les praticiens effectuent une grande partie de leurs prestations lors de visites chez les patients. L'édition des feuilles de soin électroniques chez les patients nécessite alors l'utilisation d'un micro-ordinateur portable relié au double lecteur de carte à puce. L'ensemble de cet attirail est mal aisé à transporter, il est trop lourd et, suscitant les convoitises, il provoque des vols. En outre une fois rendu à son cabinet, le praticien doit s'occuper de la transmission en différé de toutes les feuilles électroniques éditées pendant la série de visites. Ou bien, si ce praticien n'a pas pris la peine d'emmener avec lui un équipement électronique aussi important, il doit composer avec son système informatique les feuilles de soin électroniques d'une manière manuelle. La charge administrative qui repose sur ce praticien est alors tellement rédhibitoire que ce mode d'administration est rejeté et ne peut voir son développement s'épanouir.

L'invention a pour objet de remédier à ces inconvénients en proposant un système particulièrement simple pour éditer les feuilles de soin électroniques. Partant du fait que les praticiens en déplacement doivent être joints en permanence par leur téléphone mobile, dans l'invention on a eu l'idée d'adjoindre à un téléphone mobile un circuit d'édition de feuilles de soin électroniques. En pratique ce circuit d'édition comporte un double lecteur de carte à puce connectable simultanément au téléphone mobile et des moyens logiciels pour éditer cette feuille de soin électronique à partir de données prélevées dans les cartes à puce du praticien et de son patient. Ce circuit d'édition spécialisé dans un mode préféré est réalisé dans un boîtier de batterie de téléphone mobile de sorte qu'un téléphone mobile normal, ou presque normal, peut être utilisé avec une batterie normale pour une utilisation normale, ou avec un boîtier spécialisé muni des deux lecteurs pour une utilisation normale et/ou une utilisation spécialisée d'édition des feuilles de soin. En agissant ainsi on réduit les contraintes de poids, les contraintes d'encombrement et les contraintes de vols. En effet le téléphone mobile du praticien n'est alors pas plus soumis à des vols qu'un téléphone normal.

L'invention a donc pour objet un dispositif d'édition d'une feuille de soin électronique, caractérisé en ce qu'il comporte un téléphone mobile muni de deux lecteurs de cartes à puce pour recevoir respectivement une carte à puce d'un professionnel de santé et une carte à puce d'un patient, ce téléphone mobile étant en outre muni de premiers moyens logiciels pour éditer la feuille de soin à partir d'informations contenues dans ces cartes à puce.

L'invention a encore pour objet un procédé d'édition d'une feuille de soin électronique, caractérisé en ce qu'il comporte les étapes suivantes
- on introduit une carte à puce d'un professionnel de santé et une carte à puce d'un patient dans un téléphone mobile muni de deux lecteurs de cartes à puce
- on lance avec ce téléphone mobile un logiciel pour éditer la feuille de soin à partir d'informations contenues dans ces cartes à puce.

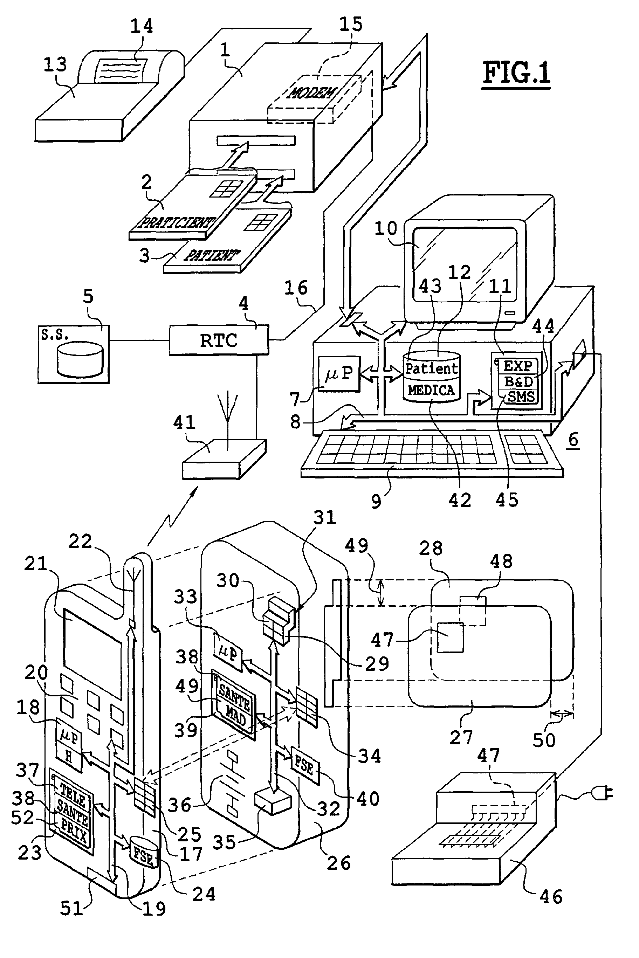

L'invention sera mieux comprise à la lecture de la description qui suit et à l'examen de la figure 1 qui l'accompagne. Celles-ci ne sont présentées qu'à titre indicatif et nullement limitatif de l'invention.

La figure 1 montre un dispositif d'édition d'une feuille de soin électronique selon l'invention. Un dispositif d'édition d'une feuille de soin électronique comporte d'une manière classique un double lecteur 1 de carte à puce pour recevoir une carte à puce 2 d'un praticien et une carte à puce 3 d'un patient. Le double lecteur 1 est muni de moyen d'édition de la feuille de soin électronique et d'un moyen de transmission de cette feuille électronique, notamment par l'intermédiaire d'un réseau téléphonique commuté 4, à un serveur 5 de données de sécurité sociale d'un organisme de sécurité sociale. Les utilisations les plus connues de ce type de système peuvent avoir pour objet de provoquer le paiement du praticien par l'organisme de sécurité sociale, au prorata des feuilles de soin éditées par ce praticien et qui ont été transmises à cet organisme.

Le double lecteur 1, pour réaliser l'édition et la transmission, peut aussi être relié à un système informatique 6 comportant une unité centrale 7 reliée par un bus 8 au lecteur 1 ainsi qu'à un clavier 9, un écran 10, une mémoire programme 11 et une mémoire de données 12. Le lecteur 1 ou bien le système informatique 6 peuvent par ailleurs être reliés à une imprimante 13 capable d'éditer une feuille de soin papier 14, ou une ordonnance, correspondant à une consultation. Pour la transmission, le système informatique 6, ou bien le lecteur 1, comportent un modem 15 relié au réseau téléphonique 4 par une ligne 16. Une édition d'une feuille de soin électronique et son traitement par le serveur 5 nécessiterait d'apporter chez chaque patient le système informatique 6, le lecteur 1 et l'imprimante 13. On comprend que ce n'est guère envisageable.

Selon l'invention, le dispositif d'édition d'une feuille de soin électronique est modifié et comporte essentiellement maintenant un téléphone mobile 17. Ce téléphone mobile 17 comporte une unité de traitement 18 reliée par un bus 19 à un clavier 20, un écran 21, un moyen d'émission réception 22 représenté schématiquement par antenne, une mémoire programme 23, une mémoire de données 24 et un connecteur fonctionnel 25 pour rentrer en relation avec un double lecteur 26 de cartes à puce. Le lecteur 26 est muni de deux lecteurs pour recevoir respectivement une carte à puce 27 d'un professionnel de santé et une carte à puce 28 d'un patient. Le lecteur 26 comporte à cet effet deux palpeurs électriques pour entrer en relation avec les puces des cartes à puce 27 et 28. Les deux palpeurs sont de préférence réalisés sous la forme d'un double palpeur 29 dont deux faces en vis-à-vis 30 et 31 portent les palpeurs et permettent de mettre les puces des cartes 27 et 28 en relation avec un bus 32 relié par ailleurs à un microprocesseur de contrôle 33, un connecteur complémentaire 34 du connecteur 25 et, de préférence un connecteur d'embase 35 du lecteur 26. Dans une variante préférée, le lecteur 26 est amovible du corps du téléphone mobile 17 et peut comporter notamment une batterie 36 utilisable pour alimenter par l'intermédiaire des connecteurs 25 et 34 le téléphone mobile 17. Dans cette version préférée l'encombrement mécanique du lecteur 26 à l'interface avec le téléphone mobile 17 est le même que celui d'une batterie classique du téléphone mobile 17. Toutefois dans ce cas, compte tenu de la présence du double lecteur 29, le lecteur 26 avec batterie peut être plus épais qu'une batterie classique.

Le téléphone mobile 17 comporte dans sa mémoire programme 23 un sous-programme 37, classique, de gestion du téléphone mobile dans le but d'assurer des télécommunications, de parole ou de données. Selon l'invention l'ensemble du téléphone mobile 17 et du lecteur 26 comportera des moyens logiciels pour éditer la feuille de soin électronique à partir d'informations contenues dans les puces des cartes à puce 27 et 28. Ces moyens logiciels peuvent prendre la forme d'un sous-programme 38 contenu dans la mémoire programme 23 ou dans une mémoire programme 39 en relation par le bus 32 avec le circuit 33. Dans ce cas le sous-programme 38, mis en oeuvre par les microprocesseurs 18 ou 33 gouverne, notamment par l'intermédiaire du circuit de commande 33, le bus 19 et le bus 32 pour prélever les informations correspondantes dans les puces des cartes 27 et 28.

Le téléphone mobile 17 possède par ailleurs un circuit d'horloge H pour incorporer une information de date dans la feuille de soin électronique. Le clavier 20 peut être utilisé pour indiquer le montant. De préférence ce montant pourra être proposé par le programme 38 s'il est forfaitaire. Dans ce cas le clavier 20 ne servira qu'à accepter la proposition faite par un menu du programme 38.

Le principe d'utilisation du dispositif selon l'invention est le suivant. Un praticien se rend avec son téléphone mobile chez un patient. Chez ce patient, après la consultation, pour éditer la feuille de soin électronique, il introduit sa carte à puce praticien 27 dans le lecteur 26 en même temps qu'il y introduit la carte à puce 28 du patient. Puis à l'aide du clavier 20 (à moins que le processus ne soit déclenché automatiquement), il provoque la mise en oeuvre du sous-programme 38 qui édite la feuille de soin électronique, qui enregistre les informations correspondantes. Cette feuille de soin électronique se présente sous la forme d'un fichier qui peut être temporairement mémorisé dans la mémoire de données 24 ou dans une mémoire de données 40 du lecteur 26. Dans ce cas, le téléphone mobile peut ne servir qu'à éditer la feuille de soin électronique.

De préférence le téléphone mobile sert également à transmettre la feuille de soin au serveur 5 de l'organisme de sécurité sociale. Dans ce but à l'aide du clavier 20 le praticien peut provoquer le déroulement d'un programme automatique d'envoi, contenu dans le sous-programme 38, pour envoyer la feuille de soin qu'il avait édité. Cet envoi au serveur 5 de l'organisme de sécurité sociale se réalise par l'intermédiaire d'une station de base 41 d'un réseau de téléphonie mobile, de préférence public, avec laquelle le téléphone mobile 17 entre en relation. Dans ce cas, le sous-programme 38, SANTE, comporte premièrement une suite d'instructions pour l'édition de la feuille de soin électronique et deuxièmement une suite d'instructions pour établir la liaison, par l'intermédiaire de la station de base 41, avec le centre serveur 5, et pour lui transmettre (de préférence après des vérifications de protocoles de transmission) la feuille de soin électronique qui a été éditée.

Il est également possible au praticien d'interroger à distance, avec son téléphone mobile 17, la base de données 12 de son système informatique 6. Dans ce but, par l'intermédiaire de la station de base 41, il entre en relation avec son modem 15, en appelant par exemple le numéro de téléphone de la ligne téléphonique 16 auquel ce dernier est raccordé. Une fois connecté, le microprocesseur 7, ou le microprocesseur 18 en mettant en oeuvre le programme 38, permettent de consulter dans la mémoire 12 une liste 42 de médicaments, avec leurs prescriptions d'usage et leurs contre-indications, ou une liste 43 de patients dans laquelle le praticien aura renseigné des informations concernant les patients qu'il connaît, et dont il veut se remémorer l'historique de santé : les dates de vaccination, les maladies précédentes, les traitements précédents et ainsi de suite. Le système peut aussi être à intelligence répartie. Dans ce cas soit le programme 38 est capable par l'intermédiaire du microprocesseur 18 de lancer toutes les opérations correspondantes, soit le microprocesseur 7 prélève dans la mémoire programme 11 un sous-programme 44 de gestion de base de données pour explorer les bases de données 42 et 43. La mémoire programme 11 peut notamment comporter un sous-programme 45 d'édition de messages au format SMS (Short Message Service - service de messages courts) qui permet de transmettre des données, par l'intermédiaire de la station de base 41 au téléphone mobile 17. Les informations correspondantes peuvent être alors affichées sur l'écran 21 de ce dernier.

Comme indiqué précédemment, le lecteur 26 peut être amovible, le téléphone mobile 17 étant alors agrémenté de deux boîtiers batterie différents : un premier boîtier batterie classique simple, non représenté pour un usage classique du téléphone mobile 17, et le boîtier batterie 26 comportant les deux lecteurs. On peut prévoir dans ce cas que la batterie 36 aura une bien plus grande capacité qu'une batterie normale.

En particulier dans le cas où le lecteur 26 est alourdi par la présence de cette batterie, le dispositif d'édition de feuilles de soin électroniques selon l'invention sera de préférence complété par un socle 46 destiné à entrer en relation par l'intermédiaire du connecteur 35 avec le système informatique du boîtier 26. Le socle 46 peut notamment être un socle de rechargement de la batterie 36 par une connexion au réseau électrique. De préférence le boîtier 46, par l'intermédiaire d'un connecteur 47, assurera une liaison fonctionnelle du lecteur 26 à un connecteur 48 du système informatique 6. Le connecteur 48 est par ailleurs relié par le bus 8 au microprocesseur 7. Dans ce cas la mémoire programme 39 pourra comporter un programme 49 MAJ de mise à jour. Le but de ce programme 49 est de prélever dans la mémoire 40 (ou dans la mémoire 24 du téléphone mobile) les informations relatives aux dernières feuilles de soin électroniques éditées et de les transférer à la mémoire de données 12 du système informatique 6. De préférence, le seul fait de poser le téléphone 17 ou le lecteur 26 sur le socle 46 provoquera la mise à jour de la mémoire 12 et/ou l'émission par le modem 15 des feuilles de soin mémorisées. Les programmes nécessaires à cette mise à jour et à cet envoi seront contenus dans la mémoire 11 ou dans la mémoire 39 (voire 23).

L'intelligence du système comportant le téléphone mobile 17, le boîtier amovible 26 et le système informatique 6 peut être répartie. Certains des programmes ou sous-programmes nécessités lorsque deux organes parmi ces trois organes sont en relation l'un avec l'autre peuvent alors être mémorisés dans l'un ou l'autre de ces deux organes, voire en partie dans chacun d'eux. On remarquera par ailleurs que l'utilisation du boîtier amovible 26 sur le socle 46 remplace le lecteur 1. Le cas échéant celui-ci peut être supprimé à condition alors de placer le modem 15 dans le système informatique 6, ou d'accepter de transmettre toutes les feuilles de soin électroniques par l'intermédiaire du téléphone mobile 17.

En pratique pour occuper le moins de place possible on a prévu que les deux palpeurs 30 et 31 seraient placés sur des faces en vis-à-vis du double palpeur 29. Dans ce cas, les cartes à puce 27 et 28 doivent être introduites avec leur puce électronique 47 et 48 respectivement en vis-à-vis l'une de l'autre. La puce 47 vient alors au contact du palpeur 30 alors que la puce 48 vient au contact du palpeur 31. Pour que ces derniers palpeurs 29 - 31 occupent le moins de place possible, on a prévu qu'ils présentent un certain décalage, vertical 49 ou horizontal 50 des deux palpeurs 30 et 31 l'un par rapport à l'autre. Dans l'exemple représenté le décalage est un décalage vertical 49. En agissant ainsi on peut montrer que l'encombrement du double palpeur 29 est réduit. De préférence le bus 32 saura interroger les puces 47 et 48 d'une manière adéquate de manière à les reconnaître même si les cartes 27 et 28 ont été interverties.

Le socle 46 peut bien entendu prendre la forme d'un connecteur monté en bout d'un câble et enfichable dans le connecteur 35 et/ou dans un connecteur 51 d'embase du téléphone mobile 17 en relation avec le bus 19. Ce connecteur enfichable serait alors, selon l'invention, un connecteur de dépôt du téléphone mobile. Dans le cas où le lecteur 26 est placé indépendamment du téléphone mobile 17 dans le socle 46, la mémoire 39 avec le sous-programme 38 sert de moyen pour faire transmettre par l'intermédiaire du système informatique 6 la feuille de soin électronique au serveur 5. Dans ce cas, toutefois, le boîtier 26 ne comporterait pas nécessairement la batterie 36.

Les connecteurs 25 et 34 complémentaires peuvent être des connecteurs spécifiques, ou être incorporé en plus de connecteurs classiques de batterie.

Selon un perfectionnement de l'invention, la mémoire programme 39 et ou la mémoire programme 23 comportent un sous programme 52, PRIX, de paiement, au praticien, du prix de la prestation par le patient, ou d'une partie de ce prix si le principal de ce prix est supporté par l'organisme de sécurité sociale. Dans ce but, après avoir édité la feuille de soin électronique, le praticien extrait les deux cartes à puce de santé, ou seulement celle du patient. Il introduit alors dans le lecteur 26 une carte à puce bancaire du patient. L'introduction de cette carte à puce bancaire provoque une reconnaissance du type de cette carte par le lecteur 26. Cette introduction permet alors de lancer automatiquement un paiement sous le contrôle du microprocesseur 18, du microprocesseur 33, ou encore du microprocesseur de la carte à puce du praticien si le procédé concerné par ce paiement le requiert. Ce paiement comporte l'indication du prix à payer par le patient à l'aide du clavier du téléphone 17 (à moins que ce prix ne soit forfaitaire). Il comporte encore le prélèvement des références bancaires d'un compte bancaire de ce patient., extraites de la carte à puce bancaire de ce patient, et éventuellement des références bancaires du praticien extraites de la carte à puce 27 de ce dernier. La composition d'un message de paiement à envoyer à un organisme bancaire, voire à l'organisme de sécurité sociale, peut ensuite être mené par la suite du programme 52. Quand le message de paiement est envoyé à l'organisme de sécurité sociale, les références bancaires du praticien ne sont pas immédiatement nécessaires : elles peuvent être fournie en correspondance par l'organisme de sécurité sociale.

## Revendications

1. Dispositif d'édition d'une feuille de soin électronique, **caractérisé en ce qu'**il comporte un téléphone mobile (17) muni de deux lecteurs (30, 31) de cartes à puce pour recevoir respectivement une carte à puce (27) d'un professionnel de santé et une carte à puce (28) d'un patient, ce téléphone mobile étant en outre muni de premiers moyens logiciels (38) pour éditer la feuille de soin à partir d'informations contenues dans ces cartes à puce.

2. Dispositif selon la revendication 1, **caractérisé en ce qu'**il comporte un socle (46) de dépôt du téléphone mobile en relation avec un système (6) informatique, et **en ce que** les premiers moyens logiciels comportent des moyens (38, 39, 49) pour transmettre la feuille de soin à ce système informatique au moment du dépôt de ce téléphone mobile sur ce socle, ce socle étant de préférence un socle de recharge de la batterie (36) du téléphone mobile.

3. Dispositif selon la revendication 2, **caractérisé en ce que** les lecteurs de cartes à puce et ou les moyens logiciels sont contenus dans un boîtier (26) amovible, notamment un boîtier de batterie, connectable (47, 48) fonctionnellement au système informatique indépendamment du téléphone mobile.

4. Dispositif selon l'une des revendications 2 ou 3, **caractérisé en ce que** le système informatique comporte un modem (15) et des deuxièmes moyens logiciels pour transmettre la feuille de soin à un serveur de données de sécurité sociale.

5. Dispositif selon l'une des revendications 1 à 4, **caractérisé en ce que** les lecteurs de cartes à puce et ou les moyens logiciels sont contenus dans un boîtier amovible de batterie connectable électriquement et fonctionnellement au téléphone mobile.

6. Dispositif selon l'une des revendications 1 à 5, **caractérisé en ce que** les moyens logiciels comportent des moyens pour transmettre la feuille de soin à un serveur (5) de données de sécurité sociale.

7. Dispositif selon l'une des revendications 1 à 6, **caractérisé en ce que** les moyens logiciels comportent des moyens pour consulter une base de données (12) mémorisée dans un site distant et accessible par ce téléphone mobile, cette base de données pouvant être une base de données de médicaments, ou une base de données de patients.

8. Dispositif selon l'une des revendications 1 à 7, **caractérisé en ce qu'**il comporte un lecteur décalé (49, 50) de cartes à puces.

9. Procédé d'édition d'une feuille de soin électronique, **caractérisé en ce qu'**il comporte les étapes suivantes
- on introduit une carte à puce (27) d'un professionnel de santé et une carte à puce (28) d'un patient dans un téléphone mobile (17) muni de deux lecteurs (30, 31) de cartes à puce
- on lance avec ce téléphone mobile un logiciel (38) pour éditer la feuille de soin à partir d'informations contenues dans ces cartes à puce.

10. Procédé selon la revendication 9, **caractérisé en ce que**
- on dépose le téléphone mobile sur un socle (46) en relation avec un système (6) informatique, et **en ce que**
- le logiciel provoque (38, 39, 49) la transmission de la feuille de soin électronique à ce système informatique au moment du dépôt de ce téléphone mobile sur ce socle,
- de préférence, avec ce socle on recharge la batterie (36) du téléphone mobile.

11. Procédé selon la revendication 10, **caractérisé en ce que**
- on connecte les lecteurs de cartes à puce fonctionnellement au système informatique indépendamment du téléphone mobile.

12. Procédé selon l'une des revendications 10 ou 11, **caractérisé en ce que**
- on transmet avec un modem (15) du système informatique la feuille de soin électronique à un serveur de données de sécurité sociale.

13. Procédé selon l'une des revendications 10 à 12, **caractérisé en ce que**
- on consulte une base de données (12) mémorisée dans un site distant et accessible par ce téléphone mobile,
- cette base de données pouvant être une base de données de médicaments, ou une base de données de patients.

14. Procédé selon l'une des revendications 10 à 13, **caractérisé en ce que**
- on introduit une carte à puce bancaire du patient dans un des deux lecteurs, et
- on provoque une session (52) de paiement du prix de la prestation fournie par le praticien en prélevant dans cette carte à puce bancaire des références bancaires d'un compte bancaire de ce patient.
